(19) Europäisches Patentamt
European Patent Office
Office européen des brevets

(11) Publication number: 0 255 224 A2

(12) EUROPEAN PATENT APPLICATION

(21) Application number: 87305551.1

(51) Int. Cl.⁴: A61K 37/02

(22) Date of filing: 23.06.87

(30) Priority: 30.06.86 US 880536

(43) Date of publication of application: 03.02.88 Bulletin 88/05

(84) Designated Contracting States: CH DE FR GB IT LI NL

(71) Applicant: MERCK & CO. INC.
126, East Lincoln Avenue P.O. Box 2000
Rahway New Jersey 07065-0900(US)

(72) Inventor: Dallob, Aimee L.
253 West 72nd street
New York New York 10023(US)
Inventor: Moore, Vernon L.
119 Sharon Garden
Woodbridge New Jersey 07095(US)
Inventor: Goldenberg, Marvin M.
721 Shackamaxon Drive
Westfield New Jersey 07090(US)

(74) Representative: Hesketh, Alan, Dr.
European Patent Department Merck & Co., Inc. Terlings Park Eastwick Road
Harlow Essex, CM20 2QR(GB)

(54) **Cyclic and bridged cyclic somatostatin analogs useful as analgesic agents.**

(57) Cyclic and bridged cyclic somatostatin analogs have been found to be effective in the treatment of peripherally mediated pain and are therefore useful as analgesic agents.

EP 0 255 224 A2

# CYCLIC AND BRIDGED CYCLIC SOMATOSTATIN ANALOGS USEFUL AS ANALGESIC AGENTS

## BACKGROUND OF THE INVENTION

This invention relates to cyclic and bridged cyclic somatostatin analogs which have been found to be effective in the treatment of peripherally mediated pain and are therefore useful as analgesic agents.

Cyclic and bridged cyclic somatostatin analogs are known compounds and are described in U.S. Patents 4,310,518 and 4,235,886, in European Application 83,111,747.8 and in Belgian Patent 900.089. In these U.S. Patents, the European Patent Application, and the Belgian Patent, these compounds are stated to be capable of inhibiting the release of glucagon, insulin, and growth hormone and reducing gastric secretions.

## DESCRIPTION OF THE INVENTION

It has now been found that peripherally mediated pain can be treated by the application of such compounds as are disclosed and described in U.S. Patents 4,310,518 and 4,235,886, in European Patent Application 83,111,747.8 and in Belgian Patent 900.089.

Therefore, this invention is directed toward the use of cyclic and bridged cyclic somatostatin analogs such as are disclosed and described in U.S. Patents 4,310,518 and 4,235,886, European Patent Application 83,111,747.8, and Belgian Patent 900.089 for treating peripherally mediated pain. These cyclic and bridged cyclic somatostatin analogs and the methods for their preparation disclosed and described in U.S. Patents 4,310,518 and 4,235,886, European Patent Application 83,111,747.8, and Belgian Patent 900.089 are incorporated herein by reference.

Thus, the cyclic and bridged cyclic somatostatin analog compounds that have been found to be effective analgesic agents useful to treat peripherally mediated pain according to this invention are those having the general formulae:

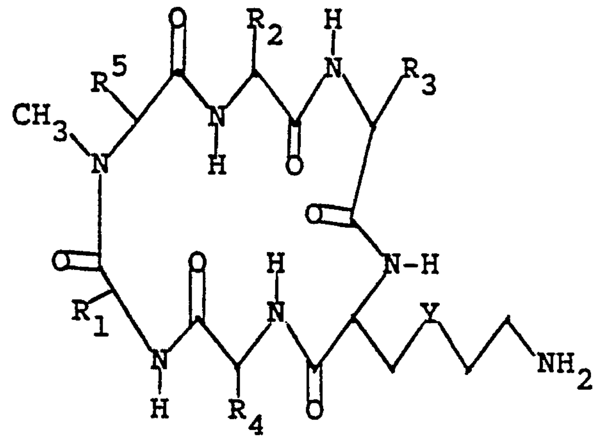

I

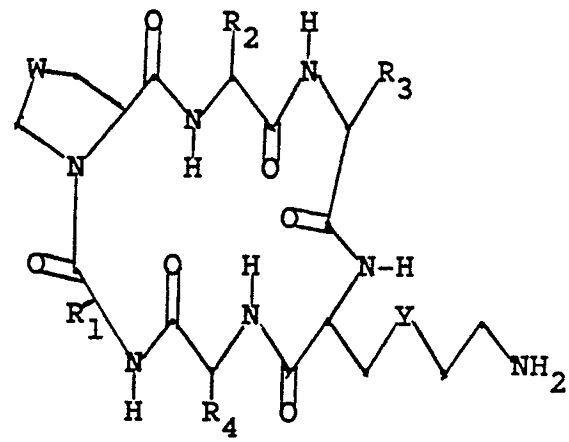

II

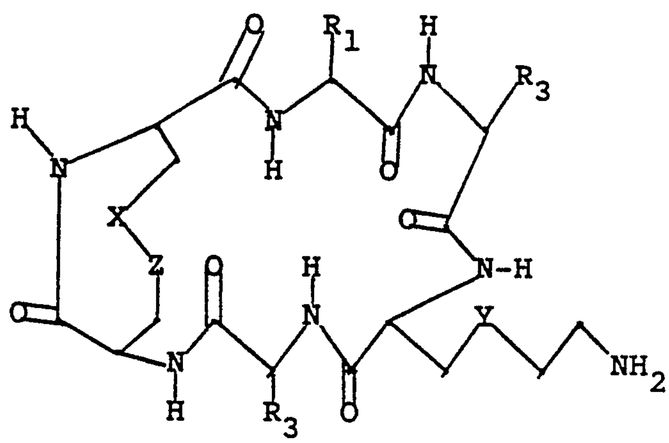

III

H-(D)Phe-Cys-Phe-(D)Trp-Lys-Thr-Cys-Thr-ol

IV

wherein in each of the compounds of Formulae I, II and III:

W is S or $(CH_2)_n$ wherein n is 0, l, or 2;

X and Z are S or $CH_2$ provided that at least one of X or Z is S;

Y is S or $(CH_2)_m$ wherein m is 0, l or 2 such that the sulfur may be in any position along the chain;

$R_1$ and $R_2$ are independently loweralkyl, benzyl, substituted benzyl where the substituent may be one or two of loweralkyl, halogen, hydroxy, amino, nitro or loweralkoxy; and loweralkyl substituted with a 5-or 6-membered heterocyclic ring;

$R_3$ is 3-indolylmethyl or substituted 3-indolylmethyl wherein the substituent may be loweralkyl, loweralkoxy, or halogen;

$R_4$ is loweralkyl, hydroxyloweralkyl, benzyl, carboxyloweralkyl, aminoloweralkyl or substituted benzyl wherein the substituent may be loweralkyl, loweralkoxy, hydroxy, halogen, amino or nitro; and

$R_5$ is loweralkyl, benzyl, or substituted benzyl wherein the substituent is loweralkyl, loweralkoxy, hydroxy, halogen amino or nitro.

In the Formulae I, II and III compounds, the term "loweralkyl" represents those alkyl groups either straight or branched chain, which have from 1-5 carbon atoms. Examples of such alkyl groups are methyl, ethyl, propyl, <u>iso</u>-propyl, butyl, <u>sec</u> -butyl, pentyl and the like.

The term "loweralkoxy" represents those alkoxy groups of from 1-5 carbon atoms, in either a straight or branched chain. Examples of such alkoxy groups are methoxy, ethoxy, propoxy, isopropoxy, butoxy, <u>tert</u>-butoxy, pentoxy and the like.

The term "halogen" or "halo" represents fluorine, chlorine, bromine and iodine.

The term "5-or 6-membered heterocyclic ring" represents those 5-and 6-membered heterocycles with 1-or 2-heteroatoms selected from oxygen, nitrogen and sulfur. Exemplary of such heterocycles is imidazole, furan, thiazole, pyrazole, pyridine and the like.

In the Formulae I, II and III compounds, there are several asymmetric centers which lead to the existence of optical isomers for such compounds. For each of the asymmetric centers of the various amino acids which make up these cyclic hexapeptides, both the D and L configurations are intended to be encompassed.

The following are representative cyclic hexapeptide analogs of somatostatin which can be respectively formed from the Formula I, II and III compounds above:

```
N-Me-Ala-Phe-Trp
     |       |
     Phe-Thr-Lys
```

Ia

```
Pro-Phe-Trp
|       |
Phe-Thr-Lys
```

IIa

```
 ____Cys-Phe-Trp
|    |       |
S    |       |
|    |       |
S    |       |
|____Cys-Thr-Lys
```

IIIa

Preferred Formula I compounds are:

1) Cyclo-(N-Me-Ala-Tyr-D-Trp-Lys-Thr-Phe)

2) Cyclo-(N-Me-Ala-Phe-D-Trp-Lys-Thr-Phe)
3) Cyclo-(N-Me-Ala-Phe-L-Trp-Lys-Thr-Phe)
4) Cyclo-(N-Me-Ala-Phe-D-Trp-Lys-Thr-p-Cl-Phe)
5) Cyclo-(N-Me-Ala-Phe-D-5-F-Trp-Lys-Thr-Phe)
6) Cyclo-(N-Me-Ala-Phe-L-5-F-Trp-Lys-Thr-Phe)
7) Cyclo-(N-Me-Ala-Phe-D-Trp-Lys-Ser-Phe)
8) Cyclo-(N-Me-Ala-Tyr-D-Trp-Lys-Val-Phe)
9) Cyclo-(N-Me-Ala-Tyr-D-Trp-Lys-Val-Trp)
10) Cyclo-(N-Me-Ala-Tyr-L-Trp-Lys-Val-Phe)
11) Cyclo-(Ser-Ala-N-Me-Phe-His-D-Trp-Lys)

Preferred Formula II compounds are:

12) Cyclo-(Pro-Tyr-D-Trp-Lys-Thr-Phe)
13) Cyclo-(Pro-Phe-D-Trp-Lys-Thr-Phe)
14) Cyclo-(Pro-Phe-L-Trp-Lys-Thr-Phe)
15) Cyclo-(Pro-Phe-D-Trp-Lys-Thr-p-Cl-Phe)
16) Cyclo-(Pro-Phe-D-5-F-Trp-Lys-Thr-Phe)
17) Cyclo-(Pro-Phe-L-5-F-Trp-Lys-Thr-Phe)
18) Cyclo-(Pro-Phe-D-Trp-Lys-Ser-Phe)

Preferred Formula III compounds are:

19) Cyclo-(Cys-Cys-Tyr-D-Trp-Lys-Thr)
20) Cyclo-(Cys-Cys-Tyr-D-Trp-Lys-Val)
21) Cyclo-(Cys-Cys-Tyr-L-Trp-Lys-Val)
22) Cyclo-(Cys-Cys-Phe-D-Trp-Lys-Thr)
23) Cyclo-(Cys-Cys-Phe-L-Trp-Lys-Thr)
24) Cyclo-(Cys-Cys-His-D-Trp-Lys-Thr)
25) Cyclo-(Cys-Cys-His-D-Trp-Lys-Val)
26) Cyclo-(Cys-Cys-Aha-Phe-D-Trp-Lys-Thr)

In the instant application several abbreviated designations are used for the amino acid components and the meaning of these abbreviated designations are given below:

| Abbreviated Designation | Amino Acid |
|---|---|
| Lys | L-lysine |
| Phe | L-phenylalanine |
| Trp | L-tryptophan |
| D-Trp | D-tryptophan |
| Thr | L-threonine |
| Aha | 7-aminoheptanoic acid |
| Tyr | L-tyrosine |
| Val | L-valine |
| Abu | L-α-aminobutyric acid |

| Abbreviated Designation | Amino Acid |
|---|---|
| Ser | L-serine |
| Asn | L-asparagine |
| Pro | L-proline |
| Asu | D- or L-aminosuberic acid |
| Cys | L-cysteine |

Treatment of peripherally mediated pain with the Formula I, II, III, and IV compounds is accomplished by providing the Formula I, II, III, and IV compounds in the form of a suitable pharmaceutical composition containing a Formula I, II, III, or IV compound or mixtures thereof as the active ingredient.

Thus, suitable pharmaceutical compositions containing the active ingredient can be in the form of creams, ointments, jellies, solutions, suspensions, nasal or inhalant sprays or drops, eye drops, pressurized or non-pressurized dispersible powders, and the like, and can be used to effectively treat warm blooded animals such as mice, rats, horses, dogs, cats, cattle, and the like, and humans. Such pharmaceutical compositions, in addition to an effective dosage amount of the active ingredient, typically include pharmaceutically acceptable carrier, adjuvants and vehicles.

For example, aqueous suspensions can be used containing the active ingredient in admixture with excipients suitable for the manufacture of aqueous suspensions. Such excipients are suspending agents, for example sodium carboxymethylcellulose, methylcellulose, hydroxypropylmethylcellulose, sodium alginate, polyvinylpyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyoxyethylene sorbitan monooleate. The aqueous suspensions may also contain one or more preservatives, for example, ethyl, or n-propyl, p-hydroxybenzoate, one or more coloring agents, one or more flavoring agents, and one or more sweetening agents, such as sucrose or saccharin.

Oily suspension can also be formulated by suspending the active ingredient in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. Sweetening agents such as those set forth above, and flavoring agents may be added to provide a palatable oral preparation. These compositions may be preserved by the addition of an antioxidant such as ascorbic acid.

Pressurized or non-pressurized dispersible powders and granules suitable for preparation of an aqueous suspension by the addition of water provide the active ingredient in admixture with a dispersing or wetting agent, suspending agent and one or more preservatives can be employed. Suitable dispersing or wetting agents and suspending agents are exemplified by those already mentioned above. Additional excipients, for example, sweetening, flavoring and coloring agents, may also be included.

The pharmaceutical composition of the present invention may also be in the form of oil-in-water emulsions. The oily phase may be a vegetable oil, for example olive oil or arachis oils, or a mineral oil, for example liquid paraffin or mixtures of these. Suitable emulsifying agents may be naturally-occurring gums, for example gum acacia or gum tragacanth, naturally-occurring phosphatides, for example soy bean, lecithin, and esters or partial esters derived from fatty acids and hexitol anhydrides, for example sorbitan monooleate, and condensation products of the said partial esters with ethylene oxide, for example polyoxyethylene sorbitan monooleate. The emulsions may also contain sweetening and flavoring agents.

The pharmaceutical compositions can be in the form of an oleagenous suspension. This suspension can be formulated according to the known art using suitable dispersing or wetting agents and suspending agents which have been mentioned above.

For topical use, creams, ointments, jellies, solutions or suspensions, etc., containing the anti-inflammatory agents are employed.

The amount of active ingredient; i.e. the Formula I, II, III, or IV compound or mixtures thereof, for use in the present compositions will vary depending, for example, on the condition being treated and the size and kind of mammal. Generally speaking, the active ingredient can be employed in any amount known to treat peripherally mediated pain as well as at doses one-fifth to one-third lower than the usual amounts for multiple daily applications.

For humans, typical effective amounts of active ingredient for use in unit dose compositions of the invention are about 0.001% to about 2.0% by weight of the composition, preferably about 0.1% to about 0.5% by weight of the composition. However, greater amounts can be employed if desired or prescribed.

## EXAMPLE

To demonstrate the use of the compounds of the invention as effective analgesic agents to treat peripherally mediated pain, a compound was selected as representative of the compounds of the invention and tested in rats. The compound used was compound 8) of the Formula I compounds having the formula:

8) Cyclo-(N-Me-Ala-Tyr-D-Trp-Lys-Val-Phe) which is identified below as "Form. I-8 compound".

## INTRODUCTION

Somatostatin and somatostatin analogs are believed to antagonize Substance P, an endogenous pain mediator. Therefore, Form. I-8 compound as representative of the cyclic and bridged somatostatin analogs of the invention was selected and tested for analgesic activity in a model of hyperalgesia. Brewer's yeast-induced hyperalgesia is a known standard test for peripherally-acting analgesics, [see, for example, L. A. Randall, et al., Arch. Int. Pharmacodyn. , 111, 409(1957), C. A. Winter, et al., J. Pharmac. Exp. Ther., 150, 165(1965), A. Rackam, et al. Prostaglandin, 25, 193(1983), and P. Davies, et al. Ann. Rev. Immunol., 2, 335(1984)]. Both indomethacin and aspirin are effective analgesic agents in this assay.

## METHODS

### Yeast-induced Hyperalgesia

Female Sprague-Dawley weanling rats (35-50 g; Taconic Farms) were fasted overnight and arranged in groups of ten. Hyperalgesia was induced in the right hindpaw by a subplantar injection of 0.1 ml of a 5 percent suspension of brewer's yeast. Hyperalgesia was measured by applying pressure to the plantar surface of the hindpaw by means of a compressed air driven piston with a 2 mm tip to obtain the vocalization threshold. Thresholds were obtained 3 hours after paw injections. Form. I-8 compound, suspended at various doses in 0.5% methylcellulose, was administered perorally (p.o.) or intraperitoneally (i.p.) in a volume of 0.10 ml, 2 hours after yeast injection. Control groups received the vehicle alone. For each compound treatment group, animals with response pressures in the inflamed paw greater than 25 mm Hg were considered to be analgesic. The results obtained are shown in Table I below.

TABLE I

Effect of Form. I-8 Compound on

Brewer's Yeast-Induced Hyperalgesia in Rats

| Route | Dose+ (mg/kg) | % Analgesia 3 Hours* | 4 Hours |
|---|---|---|---|
| i.p. | 0.1 | 0 | 0 |
| | 1.0 | 10 | 30 |
| | 10.0 | 10 | 10 |
| p.o. | 0.1 | 0 | 0 |
| | 1.0 | 0 | 10 |
| | 10.0 | 70 | 50 |
| p.o. | 0.3 | 40 | 30 |
| | 1.0 | 40 | 40 |
| | 3.0 | 40 | 0 |
| | 10.0 | 60 | 0 |
| | 30.0 | 60 | 10 |
| p.o. | 0.3 | 40 | 20 |
| | 1.0 | 60 | 30 |
| | 3.0 | 50 | 0 |
| | 10.0 | 40 | 0 |
| | 30.0 | 30 | 0 |

+ n = 9-10 mice/dose

* Time after yeast injection into paw.

Brewer's Yeast-Induced Hyperalgesia in Rats

As shown in Table I, Form. I-8 compound was relatively inactive i.p., but exhibited analgesic activity when administered perorally. Form. I-8 compound significantly inhibited yeast-induced hyperalgesia at doses of 0.3 to 30 mg/kg p.o., but no dose-response was seen. The compound was more effective one hour after administration than at two hours. No effect of the compound was seen on the contralateral (non-treated) paw, indicating that the compound is not working centrally to exert its analgesic effects in this assay. Thus, Form. I-8 compound exhibits significant activity against yeast-induced hyperalgesia.

## Claims

1. The use of a compound having the Formulae:

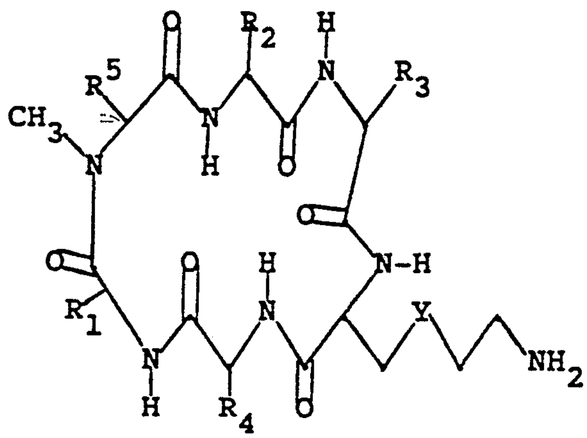

I

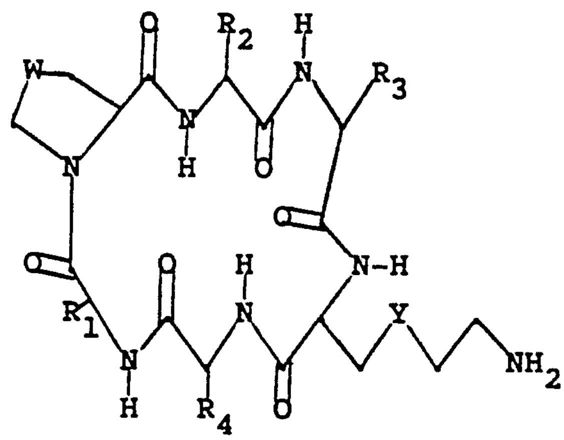

II

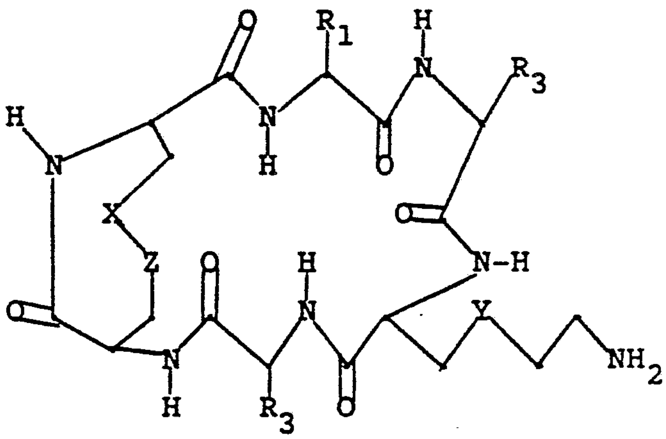

III

H-(D)Phe-Cys-Phe-(D)Trp-Lys-Thr-Cys-Thr-ol

IV

wherein in each of the compounds of Formulae I, II and III:
W is S or $(CH_2)_n$ wherein n is 0, l, or 2;
X and Z are S or $CH_2$ provided that at least one of X or Z is S;
Y is S or $(CH_2)_m$ wherein m is 0, 1 or 2 such that the sulfur may be in any position along the chain;
$R_1$ and $R_2$ are independently loweralkyl, benzyl, substituted benzyl where the substituent may be one or two of loweralkyl, halogen, hydroxy, amino, nitro or loweralkoxy; and loweralkyl substituted with a 5-or 6-membered heterocyclic ring;
$R_3$ is 3-indolylmethyl or substituted 3-indolylmethyl wherein the substituent may be loweralkyl, loweralkoxy, or halogen;
$R_4$ is loweralkyl, hydroxyloweralkyl, benzyl, carboxyloweralkyl, aminoloweralkyl or substituted benzyl wherein the substituent may be loweralkyl, loweralkoxy, hydroxy, halogen, amino or nitro; and
$R_5$ is loweralkyl, benzyl, or substituted benzyl wherein the substituent is loweralkyl, loweralkoxy, hydroxy, halogen amino or nitro; for the preparation of a medicament useful for treating peripherally mediated pain.

2. The use as claimed in Claim 1 wherein said Formula I compound is a member of the group:

Cyclo-(N-Me-Ala-Tyr-D-Trp-Lys-Thr-Phe);
Cyclo-(N-Me-Ala-Phe-D-Trp-Lys-Thr-Phe);
Cyclo-(N-Me-Ala-Phe-L-Trp-Lys-Thr-Phe);
Cyclo-(N-Me-Ala-Phe-D-Trp-Lys-Thr-p-Cl-Phe);
Cyclo-(N-Me-Ala-Phe-D-5-F-Trp-Lys-Thr-Phe);
Cyclo-(N-Me-Ala-Phe-L-5-F-Trp-Lys-Thr-Phe);
Cyclo-(N-Me-Ala-Phe-D-Trp-Lys-Ser-Phe);
Cyclo-(N-Me-Ala-Tyr-D-Trp-Lys-Val-Phe);
Cyclo-(N-Me-Ala-Tyr-D-Trp-Lys-Val-Trp);
Cyclo-(N-Me-Ala-Tyr-L-Trp-Lys-Val-Phe); and,
Cyclo-(Ser-Ala-N-Me-Phe-His-D-Trp-Lys);

said Formula II compound is a member of the group:

Cyclo-(Pro-Tyr-D-Trp-Lys-Thr-Phe);
Cyclo-(Pro-Phe-D-Trp-Lys-Thr-Phe);
Cyclo-(Pro-Phe-L-Trp-Lys-Thr-Phe);
Cyclo-(Pro-Phe-D-Trp-Lys-Thr-p-Cl-Phe);
Cyclo-(Pro-Phe-D-5-F-Trp-Lys-Thr-Phe);
Cyclo-(Pro-Phe-L-5-F-Trp-Lys-Thr-Phe); and,
Cyclo-(Pro-Phe-D-Trp-Lys-Ser-Phe); and,

said Formula III compound is a member of the group:

Cyclo-(Cys-Cys-Tyr-D-Trp-Lys-Thr);

Cyclo-(Cys-Cys-Tyr-D-Trp-Lys-Val);

Cyclo-(Cys-Cys-Tyr-L-Trp-Lys-Val);

Cyclo-(Cys-Cys-Phe-D-Trp-Lys-Thr);

Cyclo-(Cys-Cys-Phe-L-Trp-Lys-Thr);

Cyclo-(Cys-Cys-His-D-Trp-Lys-Thr);

Cyclo-(Cys-Cys-His-D-Trp-Lys-Val); and,

Cyclo-(Cys-Cys-Aha-Phe-D-Trp-Lys-Thr).